(19) Europäisches Patentamt
European Patent Office
Office européen des brevets

(11) Publication number: **0 357 097 A1**

# (12) EUROPEAN PATENT APPLICATION

(21) Application number: 89201762.5

(22) Date of filing: 03.07.89

(51) Int. Cl.5: C07C 335/26 , A01N 47/30

(30) Priority: 04.07.88 GB 8815882

(43) Date of publication of application: 07.03.90 Bulletin 90/10

(84) Designated Contracting States: BE CH DE ES FR GB IT LI NL

(71) Applicant: SHELL INTERNATIONALE RESEARCH MAATSCHAPPIJ B.V.
Carel van Bylandtlaan 30
NL-2596 HR Den Haag(NL)

(72) Inventor: Verbrugge, Pieter Adriaan
Badhuisweg 3
NL-1031 CM Amsterdam(NL)
Inventor: De Waal, Jannetje
Badhuisweg 3
NL-1031 CM Amsterdam(NL)

(74) Representative: Hunter, Keith Roger Ian et al
4 York Road
London SE1 7NA(GB)

## (54) Benzoylthiourea compounds.

(57) The invention provides benzoylthiourea compounds of general formula

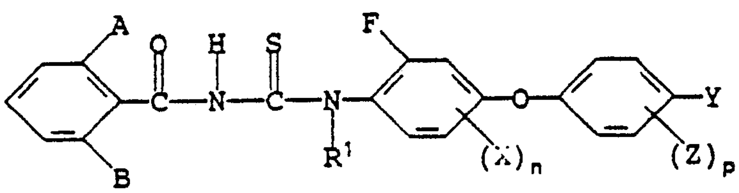

(I)

wherein each of A, B, X and Z independently represents a fluorine or chlorine atom; n is 0, 1, 2 or 3; p is 0 or 1; Y represents a haloalkyl group of up to 6 carbon atoms; and $R^1$ represents a hydrogen atom or the group $-SNR^2R^3$ in which $R^2$ represents an alkyl group of up to 6 carbon atoms and $R^3$ represents an alkyl group of up to 6 carbon atoms or a group of formula $-COR^4$ or $-COOR^4$ in which $R^4$ represents an alkyl group of up to 6 carbon atoms, or $R^2$ and $R^3$ together represent an alkylene group having 4 or 5 carbon atoms and optionally substituted by an alkoxycarbonyl group having up to 6 carbon atoms in the alkyl moiety; processes for their preparation; their use as pesticides, particularly as insecticides and acaricides; and their use as intermediates in the preparation of benzoylureas.

EP 0 357 097 A1

## BENZOYLTHIOUREA COMPOUNDS

This invention relates to benzoylthiourea compounds, to processes for their preparation, to their use as pesticides, particularly as insecticides and acaricides, and to their use as chemical intermediates.

UK Patent Specification No. 1,324,293 discloses a broad class of urea and thiourea derivatives having insecticidal activity, including the commercial compound diflubenzuron, N-(2,6-difluorobenzoyl)-N′-(4-chlorophenyl)urea.

EP-B-161 019 provides a class of benzoylurea compounds of general formula:-

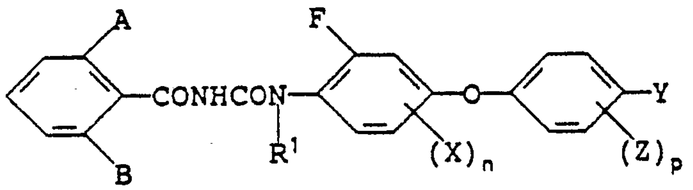
(IV)

wherein each of A, B, X and Z independently represents a fluorine or chlorine atom; n is 0, 1, 2 or 3; p is 0 or 1; Y represents a haloalkyl group of up to 6 carbon atoms; and $R^1$ represents a hydrogen atom or the group $-SNR^2R^3$ in which $R^2$ represents an alkyl group of up to 6 carbon atoms and $R^3$ represents an alkyl group of up to 6 carbon atoms or a group of formula $-COR^4$ or $-COOR^4$ in which $R^4$ represents an alkyl group of up to 6 carbon atoms, or $R^2$ and $R^3$ together represent an alkylene group having 4 or 5 carbon atoms and optionally substituted by an alkoxycarbonyl group having up to 6 carbon atoms in the alkyl moiety. These compounds are distinguished from earlier benzoyl ureas by possessing acaricidal activity in addition to insecticidal activity. This class of compounds includes the compound flufenoxuron, N-(2,6-difluorobenzoyl)-N′-(2-fluoro-4-[2-chloro-4-(trifluoromethyl)phenoxy]phenyl urea.

The compounds of formula (IV) are prepared by a process which comprises reacting a compound of the general formula:-

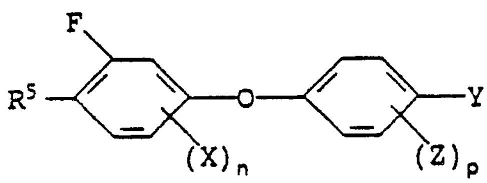

with a compound of the general formula:-

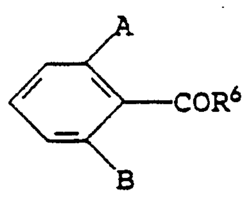

in which A, B, X, Y, Z, n and p have the meanings given above and either $R^5$ represents an $-NHR^1$ group in which $R^1$ has the meaning given above, and $R^6$ represents an -NCO group, or $R^5$ represents an -NCO group and $R^6$ represents an $-NH_2$ group.

A novel class of benzoylthioureas has now been discovered having application as intermediates in a novel synthesis route to the above compounds of formula IV, and these compounds also surprisingly possess both insecticidal and acaricidal activity.

According to the present invention therefore there are provided benzoylthiourea compounds of general formula

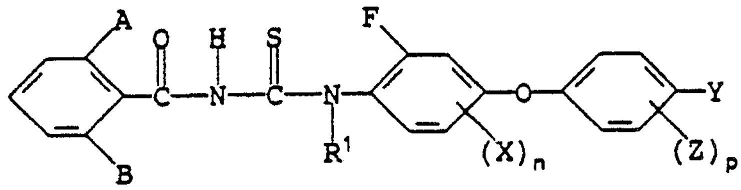

(I)

wherein each of A, B, X and Z independently represents a fluorine or chlorine atom; n is 0, 1, 2 or 3; p is 0 or 1; Y represents a haloalkyl group of up to 6 carbon atoms; and $R^1$ represents a hydrogen atom or the group $-SNR^2R^3$ in which $R^2$ represents an alkyl group of up to 6 carbon atoms and $R^3$ represents an alkyl group of up to 6 carbon atoms or a group of formula $-COR^4$ or $-COOR^4$ in which $R^4$ represents an alkyl group of up to 6 carbon atoms, or $R^2$ and $R^3$ together represent an alkylene group having 4 or 5 carbon atoms and optionally substituted by an alkoxycarbonyl group having up to 6 carbon atoms in the alkyl moiety.

Except where otherwise stated herein, any alkyl or haloalkyl group preferably has up to 4 carbon atoms. A preferred alkyl group is methyl, and a preferred haloalkyl group is trifluoromethyl. Any alkylene moiety is preferably a tetramethylene or pentamethylene group. Halogen atoms may be fluorine, chlorine, bromine or iodine atoms, with fluorine and chlorine being prefered. When n or p is greater than 1, the substituents X or Z present may be the same or different.

Preferably A and B either both represent a chlorine atom or, preferably, a fluorine atom.

Preferably X represents a fluorine or chlorine atom. Thus typically $(X)_n$ may be 3 fluorine atoms, 2 fluorine atoms and a chlorine atom, or a fluorine atom and two chlorine atoms. Most preferably X represents fluorine and n is 3, 2, 1, or, especially, 0.

Preferably Y represents a trifluoromethyl group.

Preferably Z represents a chlorine atom. Preferably p is 0 or 1. Most preferably Z represents a chlorine atom and p is 1; such a chlorine atom is preferably in the position ortho to the oxygen linkage.

$R^1$ represents a hydrogen atom or a group of formula $-S.NR^2R^3$. Preferably $R^2$ represents a methyl group. Preferably $R^3$ represents $-COOR^4$.

Preferred are compounds of the formula I in which $R^1$ represents a hydrogen atom.

The invention also provides a process for the preparation of a compound of general formula I, which process comprises reacting a compound of general formula:-

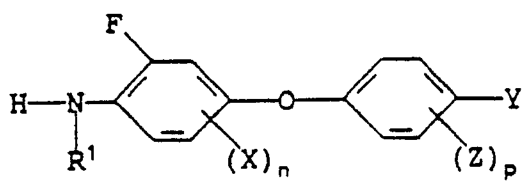

(II)

with a compound of general formula:-

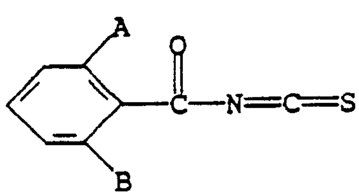

(III)

in which A, B, X, Y, Z, $R^1$, n and p are as defined above.

The reaction is suitably carried out in the presence of an inert solvent. Suitable solvents are aromatic solvents such as benzene, toluene, xylene, or chlorobenzene, hydrocarbons such as petroleum fractions, chlorinated hydrocarbons such as chloroform, methylene chloride or dichloroethane, ethers such as diethyl ether, dibutyl ether, or dioxan and ketones, such as acetone and methylethylketone. Mixtures of solvents are also suitable.

The reaction may conveniently be effected at temperatures in the range from 0°C to the reflux temperature of the reaction mixture, e.g. ambient temperature. Preferably the molar ratio of isocyanate to amine is from 1:1 to 2:1. Preferably the reaction is carried out under anhydrous conditions.

The compounds of formula II are described, together with their preparation, in EP-A-161 019.

2,6-Dihalobenzoylisothiocyanate compounds of formula III may readily be prepared by reacting the appropriate 2,6-dihalobenzoylchloride with ammonium thiocyanate (ammonium rhodanide), as described in UK Patent Specification No. 1,324,293, or with an alkali metal thiocyanate, e.g. potassium or sodium thiocyanate, conveniently in the presence of a ketone solvent, such as acetone or methylethyl ketone, at a temperature in the range from ambient temperature to reflux temperature, preferably at reflux temperature.

2,6-Dichlorobenzoyl chloride and 2,6-difluorobenzoyl chloride are known materials. 2-Chloro-6-fluorobenzoyl chloride may be prepared from the known 2-chloro-6-fluorobenzoic acid in similar manner to the preparation of 2,6-difluorobenzoyl chloride from 2,6-difluorobenzoic acid.

Compounds of general formula I may readily be oxidised to yield the corresponding benzoylurea compounds of general formula IV above, viz. the compounds of EP-B-161 019.

Accordingly the present invention also provides the use of a compound of formula I as defined above in a process for the preparation of a benzoylurea compound of general formula IV

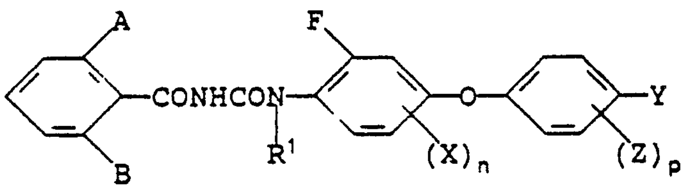

(IV)

wherein A, B, X, Y, Z, $R^1$ n and p are as defined above.

Oxidation of compounds of formula I to compounds of formula IV may conveniently be achieved, for example, using sodium hypochlorite in alcoholic medium, e.g. tertiary butyl alcohol, conveniently at 35°C to 40°C, using silver nitrate in a two-solvent system, e.g. water/ethylacetate, conveniently at ambient temperature, or preferably, using iodine and dimethyl sulphoxide, at a temperature in the range 40°C, to 100°C, preferably in the range 70°C to 90°C, conveniently at about 80°C.

Compounds of general formula I exhibit pesticidal, particularly insecticidal and acaricidal, activity. Accordingly the invention also provides a pesticidal composition comprising a compound of general formula I in association with at least one inert carrier therefor. The invention further provides a method of combating pests at a locus, which comprises applying to the locus a pesticidal compound or composition according to the invention.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating pesticidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition

according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing °-10% w of active ingredient. Granules are usually prepared to have a size between 10 and 100 BS mesh (1.676 - 0.152 mm), and may be manufactured by agglomeration or impregnation techniques. Generally, granules will contain °-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing pesticidal, herbicidal, or fungicidal properties. The compounds of the invention are especially useful when applied in admixture with other insecticides, especially organophosphates and pyrethroids. Mixtures with the commercial products fenvalerate, permethrin, cypermethrin, deltamethrin and alphamethrin are especially useful.

The invention will be further understood from the following illustrative Examples, in which Example 1 relates to the preparation of starting material, Examples 2 and 3 relate to the preparation of compounds of formula I, Examples 4 and 5 relate to oxidation of compounds of formula I to compounds of formula IV, and Examples 6 and 7 relate to pesticidal activity tests.

## Example 1

### Preparation of 2,6-difluorobenzoyl chloride

A mixture of 2,6-difluorobenzoic acid (21g, 0.133 mol) and thionyl chloride (60g, 0.465 mol) was slowly heated to reflux temperature, with vigorous evolution of gas (hydrogen chloride and sulphur dioxide). After 45 minutes at reflux temperature, evolution of gas ceased, and the mixture was kept at that temperature for a further hour, after which excess thionyl chloride was distilled off. Fractional distillation gave 2,6-difluorobenzoyl chloride (80%), bp 90°C at 30 mmHg (4 x $10^3$ Pa.s), 77°C at 25 mmHg (3.3 x $10^3$ Pa.s).

Example 2

Preparation of N-(2,6-difluorobenzoyl)-N′-(2-fluoro-4-[2-chloro-4-(trifluoromethyl)phenoxy]phenyl thiourea

Potassium thiocyanate (5g, 0.0515 mol) was dissolved in acetone (100 ml) and to the resulting solution was added 2,6-difluorobenzoyl chloride (8.5g, (97%), 0.047 mol) at ambient temperature (20°C). A precipitate was observed to form immediately. The mixture was heated at reflux temperature under stirring for 10 minutes before being cooled to 40°C.

To the resulting solution of 2,6-difluorobenzoylisothiocyanate at 40°C was added a solution of 2-fluoro-4-(2-chloro-4-[trifluoromethyl]phenoxy)aniline (14.8g, 0.047 mol) in acetone (50 ml). The temperature of the reaction mixture rose rapidly to 46°C. The reaction mixture was subsequently heated at reflux temperature for 30 minutes. Subsequent filtration (to remove potassium chloride), evaporation, dissolution of the residue in ethyl acetate, washing with water, drying ($Na_2SO_4$), filtration, evaporation, and washing with methanol (40 ml) gave N-(2,6-difluorobenzoyl)-N′-(2-fluoro-4-[2-chloro-4-(trifluoromethyl)phenoxy]phenylthiourea (19.8g, 81%)(97% pure by HPLC) m.p. 163°C. (HPLC = high performance liquid chromatography)

Example 3

Preparation of N-(2,6-dichlorobenzoyl)-N′-(2-fluoro-4-[2-chloro-4-(trifluoromethyl)phenoxy]phenyl thiourea

2,6-dichlorobenzoyl chloride (2.6g (82.5% pure), 0.0102 mol) was dissolved in acetone (25 ml), potassium thiocyanate (1.0g, 0.010 mol) was added and the mixture was heated at reflux temperature for 15 minutes. Additional potassium thiocyanate (0.5g, 0.005 mol) was added, and the mixture was stirred at ambient temperature (20°C) for a further 2 hours.

To the resulting solution containing 2,6-dichlorobenzoyl-isothiocyanate was added a solution of 2-fluoro-4-(2-chloro-4-[trifluoromethyl]phenoxy)aniline (3g, 0.010 mol) in acetone (10 ml). The temperature of the reaction mixture rose spontaneously to 22°C. After stirring for 3 hours at ambient temperature (20°C) the reaction mixture was filtered (to remove potassium chloride) and evaporated, the residue being dissolved in ethyl acetate, washed with water, dried ($Na_2SO_4$) and washed with methanol (30 ml) to give the desired product, N-(2,6-dichlorobenzoyl)-N′-(2-fluoro-4-[2-chloro-4-[2-chloro-4-(trifluoromethyl)phenoxy]-phenylthiourea, (1g). The methanol filtrate was evaporated and washed with toluene (20 ml) to give an additional amount (0.4g) of the desired product. The total desired product (1.4g, 26.4%) was 100% pure by HPLC, m.p. 167°C.

Example 4

Conversion of N-(2,6-difluorobenzoyl)-N′-(2-fluoro-4-[2-chloro-4-(trifluoromethyl)phenoxy]phenyl thiourea to N-(2,6-difluorobenzoyl)-N -(2-fluoro-4-[2-chloro-4-(trifluoromethyl)phenoxy]phenylurea

The product of Example 2 (0.5g, 0.001 mol) was dissolved in ethyl acetate (5 ml), a solution of silver nitrate (0.35g) in water (1 ml) was added and the resulting mixture was stirred overnight (16 hours) at ambient temperature (20°C). The resulting mixture was filtered to remove black precipitate, the aqueous layer was separated off and the organic layer was dried and evaporated to yield N-(2,6-difluorobenzoyl)-N′-(2-fluoro-4-[2-chloro-4-(trifluoromethyl)phenoxy]phenyl urea (0.4g, 83%), m.p. 173-174°C.

Example 5

Conversion of N-(2,6-difluorobenzoyl)-N′-(2-fluoro-4-[2-chloro-4-(trifluoromethyl)phenoxy]phenyl thiourea to

N-(2,6-difluorobenzoyl)-N′-(2-fluoro-4-[2-chloro-4-(trifluoromethyl)phenoxy]phenyl urea

The product of Example 2 (4.25g, 0.0082 mol), iodine (0.085g) and dimethyl sulphoxide (5 ml) were heated together at 80°C with stirring for 16 hours. The resulting mixture was partially evaporated, the residue was treated with ethyl acetate and sulphur (0.2g) was filtered off. The organic filtrate was washed with water, 5% w/v aqueous sodium bicarbonate and again with water, was dried and evaporated. The residue was treated with methanol and evaporated and was then stirred with methanol (20 ml) at ambient temperature (20°C) and filtered to give N-(2,6-difluorobenzoyl)-N′-(2-fluoro-4-[2-chloro-4-(trifluoromethyl)-phenoxy]phenyl urea (2.3g, 55%) m.p. 173-174°C.

Example 6

Insecticidal Activity

The insecticidal activity of compounds of the invention was assessed, employing Egyptian cotton leafworm larvae, Spodoptera littoralis (S.l.), by the following procedure.

Solutions or suspensions of test compound were made up over a range of concentrations in water containing 10%w acetone and 0.025%w "TRITON X-100" (trade mark) surface active agent (the condensation product of ethylene oxide with an alkyl phenol). These solutions were sprayed at a rate equivalent to 340 litres per hectare ($3.4 \times 10^{-5} m^3/m^2$) onto Petri dishes containing a nutritious diet for Egyptian cotton leafworm larvae. When the spray deposit had dried, each dish was infested with ten 2nd instar larvae.

Mortality assessments were made after 7 days under laboratory conditions (23°C • 2°C, fluctuating humidity and light).

In each test a $LC_{50}$ (the dosage of active material required to kill half of the test species) for the compound was calculated from the mortality figures and compared with the corresponding $LC_{50}$ for a standard insecticide, ethyl parathion, in the same test. The results are expressed as toxicity indices thus:

$$\text{toxicity index} = \frac{LC_{50}\ (\text{parathion})}{LC_{50}\ (\text{test compound})} \times 100$$

and are set out in Table I below.

Table I

| Insecticidal Activity | |
|---|---|
| Compound of Example No. | Toxicity Index (S.l.) |
| 2 | 3400 |
| 3 | 300 |

Example 7

Acaricidal Activity

Acaricidal activity of the compound of Example 2 was assessed, employing adult female glasshouse red spider mites, Tetranychus urticae (T.u.), by the following procedure.

2 cm diameter leaf discs cut from the leaves of French bean plants were placed, underside uppermost, on 5.5 cm diameter filter papers, kept moist by a cotton wool wick dipped in water.

Each leaf disc was infested with 25 to 30 adult female mites which were removed after 6 hours, leaving about 50 eggs on each disc. Within 5 days the eggs hatched. The freshly emerged larvae on the leaf discs were sprayed with solutions of test compound made up as in Example 6 above, at a rate equivalent to 340 litres per hectare ($3.4 \times 10^{-5}$ $m^3/m^2$).

The discs were thereafter kept under normal laboratory conditions (23°C • 2°C, fluctuating humidity and 16 hours day length). After 7 days assessment was made of the number of mites emerging as adults.

From the results the $LC_{50}$ was calculated. For the compound of Example 2 the value was 0.00066%w/v.

## Claims

1. A benzoylthiourea compound of general formula

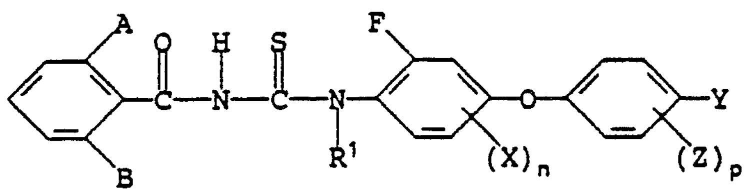

(I)

wherein each of A, B, X and Z independently represents a fluorine or chlorine atom; n is 0, 1, 2 or 3; p is 0 or 1; Y represents a haloalkyl group of up to 6 carbon atoms; and $R^1$ represents a hydrogen atom or the group $-SNR^2R^3$ in which $R^2$ represents an alkyl group of up to 6 carbon atoms and $R^3$ represents an alkyl group of up to 6 carbon atoms or a group of formula $-COR^4$ or $-COOR^4$ in which $R^4$ represents an alkyl group of up to 6 carbon atoms, or $R^2$ and $R^3$ together represent an alkylene group having 4 or 5 carbon atoms and optionally substituted by an alkoxycarbonyl group having up to 6 carbon atoms in the alkyl moiety.

2. A compound according to Claim 1 wherein Y represents a trifluoromethyl group, n is 0, p is 1 and Z represents a chlorine atom ortho to the oxygen linkage.

3. A compound according to Claim 1 or 2 wherein $R^1$ represents a hydrogen atom.

4. A compound according to Claim 1, 2 or 3 wherein A and B each represent a fluorine atom.

5. A process for the preparation of a compound of formula I as defined in any one of Claims 1 to 4, which comprises reacting a compound of general formula:-

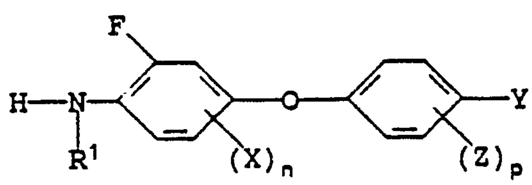

(II)

with a compound of general formula:-

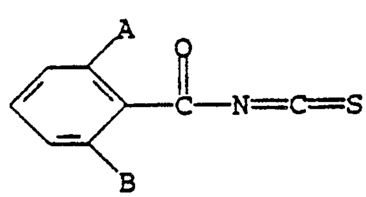

(III)

in which A, B, X, Y, Z, $R^1$, n and p are as defined in Claim 1.

6. A pesticidal composition comprising a compound as claimed in any one of Claims 1 to 4, in association with at least one inert carrier therefor.

7. A method of combating pests at a locus, which comprises applying to the locus a compound as

claimed in any one of Claims 1 to 4 or a composition as claimed in Claim 6.

8. Use of a compound of formula I as defined in any one of Claims 1 to 4 in a process for the preparation of a benzoylurea compound of general formula:-

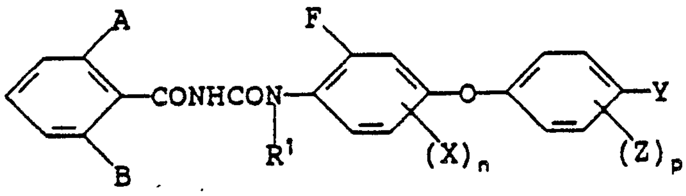

(IV)

wherein A, B, X, Y, Z, $R^1$, n and p are as defined in Claim 1.

9. Use according to Claim 8 which comprises oxidising the compound of formula I using iodine and dimethyl sulphoxide at a temperature in the range 40°C to 100°C.

European Patent Office

# EUROPEAN SEARCH REPORT

Application number: EP 89201762.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP - B1 - 0 161 019 (SHELL INTERNATIONALE RESEARCH MAATSCHAPPIJ B.V.) * Claims 1-6,8 * -- | 1-8 | C 07 C 335/26<br>A 01 N 47/30 |
| D,A | GB - A - 1 324 293 (N.V.PHILIPS' GLOEILAMPEN-FABRIEKEN) * Claims 1,75,76 * ---- | 1,5,6,8 | |

TECHNICAL FIELDS SEARCHED (Int. Cl.4)

C 07 C

The present search report has been drawn up for all claims

| Place of search | Date of completion of the search | Examiner |
|---|---|---|
| VIENNA | 11-09-1989 | HEIN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document